## Europäisches Patentamt

## European Patent Office

## Office européen des brevets

(11) Veröffentlichungsnummer : **0 094 907**
**B1**

(12) **EUROPÄISCHE PATENTSCHRIFT**

(45) Veröffentlichungstag der Patentschrift :
25.03.87

(51) Int. Cl.⁴ : **G 01 N 33/20**, G 01 N 21/35

(21) Anmeldenummer : 83810188.9

(22) Anmeldetag : 04.05.83

(54) **Verfahren zur Messung der Oxidationsgeschwindigkeit einer Metallschmelze.**

(30) Priorität : 19.05.82 CH 3126/82

(43) Veröffentlichungstag der Anmeldung :
23.11.83 Patentblatt 83/47

(45) Bekanntmachung des Hinweises auf die Patenterteilung : 25.03.87 Patentblatt 87/13

(84) Benannte Vertragsstaaten :
AT CH FR GB IT LI NL SE

(56) Entgegenhaltungen :
BE-A- 849 021
DE-A- 2 716 291
FR-A- 2 054 235

(73) Patentinhaber : SCHWEIZERISCHE ALUMINIUM AG
CH-3965 Chippis (CH)

(72) Erfinder : Silvano, Freti
Chemin de Perrelet 6
CH-1020 Renens (CH)
Erfinder : Buxmann, Kurt
Route de Sion 28
CH-3960 Sierre (CH)

## Beschreibung

Die Erfindung betrifft ein Verfahren zur Messung der Oxidationsgeschwindigkeit an der Oberfläche einer Metallschmelze, insbesondere einer Schmelze aus Aluminium oder einer Aluminiumlegierung.

Das Oxidationsverhalten von Metallschmelzen wird durch gelöste metallische Elemente, die als Verunreinigungen oder auch als Legierungskomponenten vorhanden sein können, mehr oder weniger stark beeinflusst. So führt beispielsweise die Anwesenheit von Lithium oder Magnesium in einer Aluminiumschmelze zu einer Erhöhung der Oxidationsgeschwindigkeit.

In Giessereien tritt die Oxidation von Metallschmelzen vor allem bei der Bildung von Krätze in Erscheinung ; diese kann als Folge des hierbei auftretenden Metallverlustes zu einem gewichtigen Kostenfaktor werden. Es sind deshalb Bestrebungen im Gange, die Krätzemenge auf ein Minimum herabzusetzen. Die Krätzebildung wird entscheidend durch die Oxidationsgeschwindigkeit der Metallschmelze beeinflusst, d. h. die Geschwindigkeit, mit der sich eine Oxidhaut bildet.

Die Messung der Oxidationsgeschwindigkeit einer Metallschmelze wird üblicherweise so durchgeführt, dass die Oxidhaut in unterschiedlichen Zeitabständen von der Schmelzeoberfläche abgezogen und nach dem Herauslösen der metallischen Anteile filtriert, ausgeglüht, ausgewogen und analysiert wird.

Angesichts dieser Gegebenheiten hat sich der Erfinder das Ziel gesetzt, eine Verfahren zu schaffen, mit welchem die Oxidationsgeschwindigkeit an der Oberfläche einer Metallschmelze und damit auch die Konzentration von die Oxidationsgeschwindigkeit beeinflussenden Elementen in der Metallschmelze auf einfache Weise bestimmt werden kann.

Erfindungsgemäss wird die Aufgabe dadurch gelöst, dass bei konstanter Temperatur die Oxidhaut auf der Schmelzeoberfläche abgezogen und die elektromagnetische Strahlung während der nachfolgenden Oxidation mit einem IR-Strahlungspyrometer gemessen wird, wobei die Intensitätsänderung der elektromagnetischen Strahlung als Mass für die Oxidationsgeschwindigkeit bzw. für die Konzentration gewertet wird.

Mit dem Abziehen der Oxidhaut wird kurzzeitig eine oxidfreie Metalloberfläche geschaffen. Durch die sofort wieder einsetzende Oxidation wird eine neue Oxidhaut gebildet, wodurch sich gleichzeitig das Emissionsverhalten der Metalloberfläche und damit die Intensität der von ihr ausgehenden Temperaturstrahlung ändert. Der Intensitätsverlauf der elektromagnetischen Strahlung in Funktion der Zeit spiegelt damit zumindest qualitativ die Oxidationsgeschwindigkeit an der Metalloberfläche wider. Quantitative Ergebnisse lassen sich mit Hilfe von Eichmessungen erzielen. Auf gleiche Weise können die Konzentrationen von in der Metallschmelze enthaltenen, die Oxidationsgeschwindigkeit beeinflussenden Elementen bestimmt werden.

Das erfindungsgemässe Verfahren ist insbesondere geeignet für Untersuchungen zum Kurzzeit-Oxidationsverhalten von Schmelzeoberflächen von Aluminium und Aluminiumlegierungen, aber auch von anderen Metallen wie beispielsweise Kupfer oder Stahl. Das Verfahren hat sich als besonders vorteilhaft bei der Bestimmung des Lithiumgehalts in Aluminiumschmelzen herausgestellt.

Weitere Vorteile, Merkmale und Einzelheiten der Erfindung ergeben sich aus der nachfolgenden Beschreibung eines bevorzugten Ausführungsbeispiels sowie anhand der Zeichnung ; diese zeigt in

Figur 1 eine schematisierte Darstellung einer Messeinrichtung ;

Figur 2 eine schematisierte Darstellung von Messergebnissen.

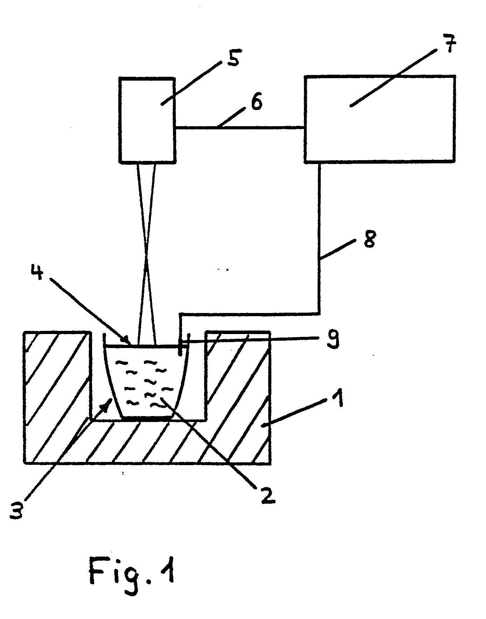

Eine Messeinrichtung weist gemäss Fig. 1 einen Ofen 1 mit einer Leistung von beispielsweise 2 kW auf, dessen Innenraum einen mit Metallschmelze 2 gefüllten Tiegel 3 aufnimmt. Auf die Schmelzeoberfläche 4 ist ein IR-Strahlungspyrometer 5 mit einer Bandbreite von beispielsweise 2,8-3,3 µm gerichtet, welches über einer Leitung 6 mit einem Aufzeichnungsgerät 7 in Verbindung steht. An das Aufzeichnungsgerät 7 ist über eine weitere Leitung 8 ein Thermoelement 9 zur Kontrolle der Oberflächentemperatur der Metallschmelze 2 angeschlossen. In der Zeichnung nicht dargestellt ist ein zum Abziehen der Oxidhaut benötiger Schaber, welcher jeweils auf derselben Temperatur wie die Metallschmelze gehalten wird, damit die Messergebnisse nicht durch Temperaturveränderungen während des Abziehens der Oxidnaut verfälscht werden.

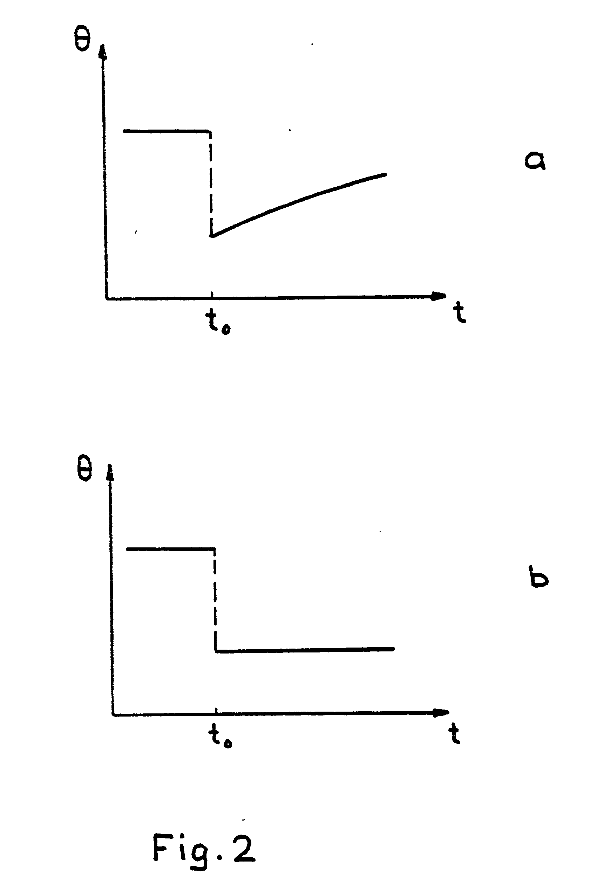

In Fig. 2 sind schematisch die Versuchsergebnisse an Aluminiumschmelzen mit einem Gehalt an Lithium von 3 ppm (a) bzw. < 0,5 ppm (b) dargestellt. Bei Anwesenheit von Lithium in der Metallschmelze steigt die am Pyrometerausgang gemessene elektrische Spannung $\theta$ nach dem Abziehen der Oxidhaut von der Schmelzeoberfläche bei $t_0$ in Folge der raschen Neuformierung der Oxidhaut sofort wieder stark an. Ist dagegen praktisch kein Lithium in der Schmelze vorhanden, so wächst die Oxidhaut bedeutend langsamer, d. h. der Anstieg der am Pyromete-rausgang gemessenen elektrischen Spannung $\theta$ zeigt nach dem Abziehen der Oxidhaut einen entsprechend flacheren Verlauf.

## Patentansprüche

1. Verfahren zur Messung der Oxidationsgeschwindigkeit an der Oberfläche einer Metallschmelze, insbesondere einer Schmelze aus Aluminium oder einer Aluminiumlegierung, dadurch gekennzeichnet, dass bei konstanter Temperatur die Oxidhaut auf der Schmelzeoberfläche abgezogen und die elektromagnetische Strahlung während der nachfolgenden Oxidation mit einem IR-Strahlungspyrometer gemessen wird, wobei die Intensitätsänderung der elektromagnetischen Strahlung als Mass für die Oxidationsgeschwindigkeit gewertet wird.

2. Verfahren zur Messung der Konzentration von die Oxidationsgeschwindigkeit beeinflussenden Elementen in einer Metallschmelze, insbesondere zur Bestimmung des Lithiumgehalts in einer Aluminiumschmelze, dadurch gekennzeichnet, dass bei konstanter Temperatur die Oxidhaut auf der Oberfläche der Metallschmelze abgezogen und die elektromagnetische Strahlung während der nachfolgenden Oxidation mit einem IR-Strahlungspyrometer gemessen wird, wobei die Intensitätsänderung der elektromagnetischen Strahlung als Mass für die Konzentration gewertet wird.

## Claims

1. A method of measurement of the rate of oxidation at the surface of a metal melt, in particular a melt of aluminium or aluminium alloy, characterized in that at constant temperature the skin of oxide is drawn off the surface of the melt and the electromagnetic radiation during the succeeding oxidation is measured by an infrared radiation pyrometer, the change in intensity of the electromagnetic radiation being taken as a measure of the rate of oxidation.

2. A method of measurement of the concentration of elements in a metal melt, which influence the rate of oxidation, in particular for the determination of the lithium content in an aluminium melt, characterized in that at constant temperature the skin of oxide is drawn off the surface of the metal melt and the electromagnetic radiation during the succeeding oxidation is measured by an infrared radiation pyrometer, the change in intensity of the electromagnetic radiation being taken as a measure of the concentration.

## Revendications

1. Procédé pour mesurer la vitesse d'oxydation à la surface d'un bain de métal, en particulier d'un bain d'aluminium ou d'alliage d'aluminium, caractérisé en ce que, à température constante, on retire la peau oxydée de la surface du bain puis on mesure le rayonnement électromagnétique pendant l'oxydation consécutive, au moyen d'un pyromètre à rayonnement infrarouge, et on exploite la variation de l'intensité du rayonnement électromagnétique comme mesure de la vitesse d'oxydation.

2. Procédé pour mesurer la concentration d'éléments influençant la vitesse d'oxydation dans un bain de métal, en particulier pour déterminer la teneur en lithium dans un bain d'aluminium, caractérisé en ce que, à température constante, on retire la peau oxydée de la surface du bain puis on mesure le rayonnement électromagnétique pendant l'oxydation consécutive, au moyen d'un pyromètre à rayonnement infrarouge, et on exploite la variation de l'intensité du rayonnement électromagnétique comme mesure de la concentration.

Fig. 1

Fig.2